Europäisches Patentamt

European Patent Office

Office européen des brevets

(11) Publication number: **0 075 039**
**B1**

## (12) EUROPEAN PATENT SPECIFICATION

(45) Date of publication of patent specification: **17.12.86**

(21) Application number: **81107551.4**

(22) Date of filing: **23.09.81**

(51) Int. Cl.⁴: **F 16 K 15/14, A 61 M 25/00**

(54) Check valve for use with intravenous administration sets.

(43) Date of publication of application:
**30.03.83 Bulletin 83/13**

(45) Publication of the grant of the patent:
**17.12.86 Bulletin 86/51**

(84) Designated Contracting States:
**AT BE CH DE FR GB IT LI LU NL SE**

(56) References cited:
**DE-A-2 829 583**
**DE-A-3 014 470**
**FR-A-2 382 998**
**GB-A-2 027 168**
**US-A-3 196 890**
**US-A-3 886 937**
**US-A-3 889 710**
**US-A-4 103 686**
**US-A-4 219 022**

(73) Proprietor: **Intermedicat GmbH**
**Gerliswilstrasse 45**
**CH-6020 Emmenbrücke (CH)**

(72) Inventor: **Raines, Kenneth**
**1727 Markham Drive**
**Bethlehem Pennsylvania 18017 (US)**

(74) Representative: **von Kreisler, Alek, Dipl.-Chem.**
**et al**
**Deichmannhaus am Hauptbahnhof**
**D-5000 Köln 1 (DE)**

Courier Press, Leamington Spa, England.

## Description

The present invention relates to a backflow preventing check valve for use in intravenous administration sets.

There are several devices presently available which concern intravenous administration sets. An example of such devices is shown in US—A—3,886,937, and in GB—A—2.027.168.

However, these known devices usually include a check valve which is expensive and not entirely reliable. These problems arise due to the complex nature of any backflow preventing element used in the check valve. Such backflow preventing elements used in the prior art often include legs, spacers, or the like located on the surfaces of the backflow preventing element. Because of these extra elements on the backflow preventing element, exotic manufacturing techniques are often required, thereby increasing the overall cost of the check valve, while reducing the overall reliability of that check valve.

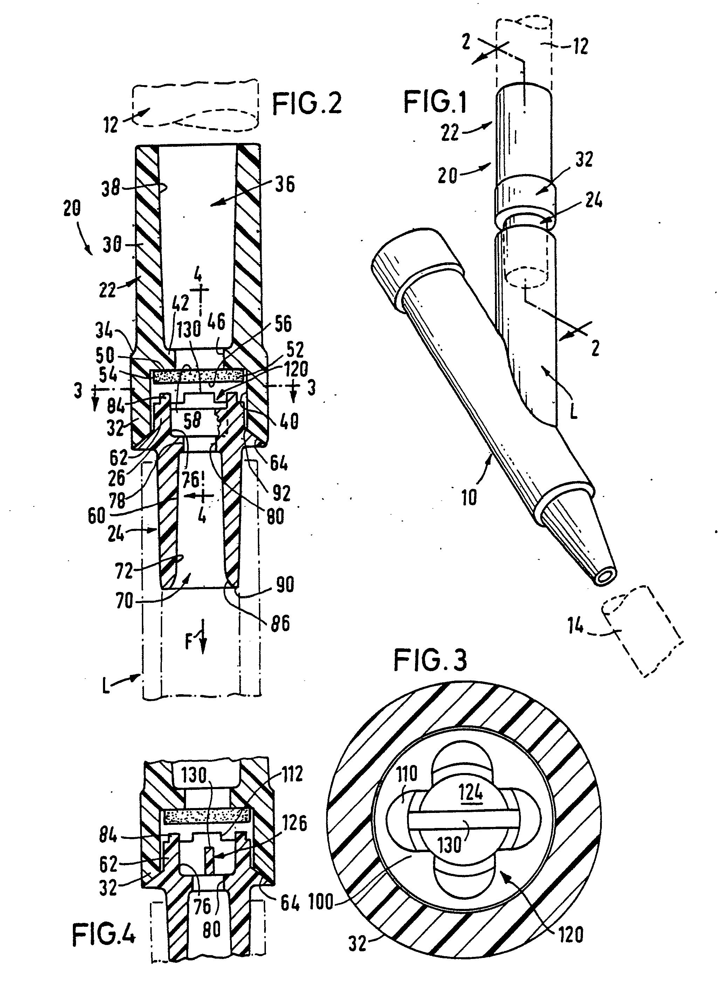

A check valve in accordance with the prior art portion of claim 1 is known from FR—A—2.382.998, figure 1.

The check valve disclosed in US—A—3.889.710 includes a female hub and a male hub sonically welded together. Flow passages are defined through the hubs to be positioned with respect to each other so that fluid can flow through the device when desired.

A flow control or obturator disc is located between the two hubs to permit flow in one direction, but to prevent flow in the other direction, i.e., backflow. The disc is circular in peripheral outline and has unobstructed flat surfaces thereon. A disc abutment means is mounted on the female hub to prevent the disc from becoming dislodged and moving into the female hub flow passage. Flow bypass paths are defined in the female hub element so that flow can bypass the flow control disc when fluid communication between the male and female hub flow passages is desired.

The check valve disc known from US—A—3.889.710 is manufactured by techniques such as simple die cutting, stamping or the like. The check valve disc is preferably rubber, or other substance having a natural resiliency. The natural resiliency produces a positive backflow prevention, thereby further increasing the reliability of the check valve.

The backflow check valve essentially prevents flow from a primary set until a secondary bottle is emptied when used in conjunction with a Y-injection site.

It is the main object of the present invention as claimed in claim 1 to provide a check valve of low costs and high reliability.

Particular embodiments of the invention are set out in the dependent Claims.

Other objects and advantages which will become subsequently apparent reside in the details of construction and operation as more fully hereinafter described and claimed, reference being had to the accompanying drawings forming part hereof.

Fig. 1 is a perspective of a portion of an intravenous administration set which includes the backflow preventing check valve embodying the teachings of the present invention.

Fig. 2 is a view taken along line 2—2 of Fig. 1.

Fig. 3 is a view taken along line 3—3 of Fig. 2.

Fig. 4 is a view taken along line 4—4 of Fig. 2.

Shown in Fig. 1 is a Y-injection site means 10 inserted between tubing 12 and tubing 14 with a backflow preventing check valve 20 connecting tubing 12 to the injection site 10.

The backflow check valve is the subject of the present disclosure, and is best shown in Fig. 2 to include a female hub 22 and a male hub 24. The hubs 22 and 24 are connected together by a sonic weld 26.

The female hub includes a cylindrical trunk section 30 and a cylindrical coupling section 32 having an outer diameter larger than the outer diameter of the trunk section to define a shoulder 34 at the intersection of these two sections. A bore 36 is defined longitudinally of the hub 22 to define a female luer taper 38. The coupling section 32 has a cylindrical bore 40 defined longitudinally thereof which has an inner diameter larger than the inner diameter of the bore 36 located adjacent the bore 40. A circular shoulder 42 is defined at the discontinuity formed at the intersection of the two bores 36 and 40, and a port 46 is defined centrally through the shoulder 42 to fluidly connect the two bores 36 and 40 together.

A circular flow control disc 50 rests on upper surface 52 of the shoulder 42 and has an outer diameter slightly smaller than the outer diameter of the bore 40 to define an annular gap 54 between these two elements. Preferably, the disc 50 is rubber having a specific gravity of about 0.98. A specific gravity of less than one permits the disc to float upward into the Fig. 2 occluding position, thereby aiding the closing of the check valve when flow is in the wrong direction.

The disc 50 has smooth, unobstructed first and second surfaces 56 and 58, respectively, and is essentially circular in peripheral outline. Such a shape permits easy manufacture as by stamping, die cutting, or the like, thereby providing a check valve which is far less costly than prior art devices which include flow blocking elements having legs, or other such elements on the surfaces thereof. Such prior art obtuating elements having legs, and the like, are difficult to manufacture and hence expensive and not as reliable as the disc 50.

The male hub 24 includes a cylindrical trunk section 60 and a cylindrical coupling section 62. A sealing shoulder 64 surrounds the hub adjacent the intersection of the trunk and coupling sections 60 and 62. The sealing shoulder is sonically welded to the female hub coupling section to form the sonic seal 26.

A bore 70 is defined longitudinally of the trunk section 60 and is tapered to form a male luer taper 72. A cylindrical bore 76 is defined longitudinally of the coupling section 62, and has an inner diameter larger than the inner diameter of that part of the luer taper 72 located adjacent the coupling section to define a shoulder 78 at the intersection of the two bores 70 and 76. A port 80 is defined through the shoulder 78 to fluidly couple the bores 70 and 76 together.

A cylindrical skirt portion 84 is located on the lowermost portion of the male hub 24, and a tapered portion 86 is located adjacent outer rim 90 of the hub 24.

As shown in Fig. 2, the outer diameter of the coupling section 62 is less than the inner diameter of the coupling section bore 40 to define an annular gap 92 between those two coupling sections. The tubing 12 is attached to the female hub 22, and a leg L of the Y-injection site 10 is coupled to the male hub 24.

A plurality of ribs 110 are defined on the coupling section skirt portion 84 to extend longitudinally of the male hub 24. As best shown in Figs. 3 and 4, the ribs are spaced apart circumferentially about the bore 76, and define flow passages 112 therebetween.

A disc abutment member 120 is attached to the male hub and extends across the male hub flow passage and is sized to seat the disc thereon. The member 120 encloses an open area 124 therein. The member has a disc abutment surface 130 thereon. The surface 130 contacts first surface 56 of the disc 50 when fluid flows through the check valve in the direction of arrow F in Fig. 2 with sufficient force to move the disc from the obturating position shown therefor in Fig. 2 into abutting contact with the male hub. The disc abuts the member 120 and is thus prevented from being forced into the bore 76 by the fluid passing from the female hub into the male hub in the flow direction F. When the disc abuts the male hub, fluid flows through the flow passages 112 to bypass the disc. However, when the fluid flow is in the direction opposite to the arrow F, the disc 50 abuts the shoulder 42 and occludes the port 46 and the bore 36 to prevent backflow of fluid from the male hub into the bore 36 and hence into the tubing 12 via the valve 20. The Fig. 2 position is thus a closed position for the check valve 20, however, in an intravenous application, the valve is normally open.

When fluid flow is in the direction indicated by the arrow F, the disc 50 is moved against the male hub and the abutment member 120. Fluid flows from port 46 longitudinally of the coupling section 32, then radially of that section when in contact with the disc second surface 58, then longitudinally of the coupling through the gap 54, then generally radially of the disc and coupling along the first surface 56 of the disc through bypass means 112. The fluid moves through open area 124 of the abutment member 120, then moves longitudinally of the male hub through the port 80 and into the leg L.

## Claims

1. A backflow preventing check valve (20) for use with intravenous administration sets for dispensing medical liquids comprising:

— a female hub (22) in polymer material having a flow passage (36, 40) defined therethrough, a valve seat (42) defined therein and a coupling section (32) thereon

— a male hub (24) in polymer material having a flow passage (70, 76) defined therethrough, a trunk section (60) and a coupling section (62) thereon, said flow passages (36, 40; 70, 76) being positioned so that fluid communication can be established therebetween the hub coupling sections (32, 62) being nested into each other;

— a circular flow control disc (50) with flat unobstructed upper and lower surfaces movably located within said female hub flow passage (40) and movable from a first position seated on said valve seat (42) obturating said female hub flow passage (36) to prevent backflow of fluid from said male hub (70, 76) to said female hub flow passage (36) to a second position in which the entire disc (50) is spaced from said valve seat (42) permitting flow of fluid from said female hub (22) to said male hub (24);

— bypass and abutment means (120) on said male hub (24) cooperating with the disc (50) being spaced from said valve seat (42) so that said disc (50) traverses a gap before abutting said bypass and abutment means (120), a flow passage (54) being defined between said disc (50) in its second position and said female hub flow passage (36, 40), said bypass and abutment means (120) including a plurality of skirt members (84) spaced apart circumferentially around said male hub flow passage (76) and extending longitudinally of said male hub (24), the spaces (112) between said skirt members (84) fluidly connecting said male hub flow passage (70, 76) to said female hub flow passage (36, 40) so that fluid can bypass said flow control disc (50) when said disc (50) is seated on said skirt members (84),

characterized in that the hub coupling sections (32; 62) are cylindrically shaped, in that a cross rib with a disc abutment surface (130) for preventing said disc (50) from moving into said male hub flow passage (70, 76) is attached to said male hub (24) and extends across said male hub flow passage (70; 76), and in that a radial, tapered sealing shoulder (64) surrounds the male hub (24) adjacent the intersection of the coupling section (62) and the trunk section (60), said sealing shoulder (64) being connected to a mating end surface of said female hub (22) by a sonic weld (26).

2. The check valve defined in claim 1, wherein the bypass flow paths (112) of the bypass and abutment means (120) are arcuate in transverse cross-section.

3. The check valve defined in claims 1 or 2 wherein said female hub (22) further includes a trunk section (30), the trunk section (30) and the coupling section (32) each having a bore (36, 40) defined therethrough, and a shoulder (42) located between said bores (36, 40), said shoulder (42) having a port (46) defined therethrough to fluidly connect said female hub bores (36, 40) together.

4. The check valve defined in claims 1 to 3 wherein the trunk section (60) and the coupling section (62) of the male hub (24) each having a bore (70, 76) defined therethrough, and a second shoulder (78) located between said male hub bores (70, 76), said second shoulder (78) having a port (80) defined therethrough to fluidly connect said male hub bores (70, 76) together.

5. The check valve defined in claims 3 and 4 wherein said hub trunk section bores (36, 70) are tapered and said coupling section bores (40, 76) are essentially cylindrical.

6. The check valve defined in claim 1 wherein said male hub further includes a tapered portion (86) thereon.

7. The check valve defined in claims 1 to 6 wherein said disc (50) has a specific gravity of less than 1,0, preferably 0,98.

8. The check valve defined in claim 1 to 7 wherein said disc (50) is die cut.

9. The check valve defined in claims 1 to 8 characterized in that a flow tube (12) is connected to said female hub (22) and a Y-injection site means (10) fluidly connected to said male hub (24).

**Patentansprüche**

1. Rückströmung verhinderndes Rückschlagventil (20) zur Verwendung an einer Infusionseinrichtung zur Überleitung medizinischer Flüssigkeiten, bestehend aus

— einem Hohlansatz (22) aus Polymermaterial, der einen durchgehenden Strömungskanal (36, 40), einen Ventilsitz (42) und einen Kupplungsteil (32) aufweist;

— einem Einstickansatz (24) aus Polymermaterial, der einen durchgehenden Strömungskanal (70, 76), einen Schaftteil (60) und einen Kupplungsteil (62) aufweist, wobei die Strömungskanäle (36, 40; 70, 76) so angeordnet sind, daß zwischen ihnen Fluidverbindung herstellbar ist und wobei die Kupplungsteil (32, 62) ineinandergesetzt sind;

— einer kreisförmigen Strömungsregulierscheibe (50) mit ebener, vorsprungsloser Ober- Unterfläche, die in dem Strömungskanal (40) des Hohlansatzes beweglich angeordnet und aus einer ersten gegen den Ventilsitz (42) anliegenden Position, in der sie den Strömungskanal (36) des Holansatzes absperrt, um ein Rückströmen von Fluid von den Strömungskanal (70, 76) des Einsteckansatzes zu dem Strömungskanal (36) des Hohlansatzes zu verhindern, in eine zweite Position bewegbar ist, in der die gesamte Scheibe (50) von dem Ventilsitz (42) abgehoben ist, damit Fluid von dem Hohlansatz (22) zu dem Einsteckansatz (24) strömen kann;

— Bypass- und Auflagermitteln (120) an dem Einsteckansatz (24), die mit der von dem Ventilsitz (42) abgehobenen Scheibe (50) zusammenwirken, so daß die Scheibe (50) einen Spalt durchquert, bevor sie gegen die Bypass- und Auflagermittel (120) anliegt, wobei zwischen der Scheibe (50) in ihrer zweiten Position und dem Strömungskanal (36, 40) des Hohlansatzes ein Strömungsdurchlaß (54) gebildet wird, wobei die Bypass- und Auflagermittel (120) mehrere Randelemente (84) aufweisen, die mit Abstand umfangsmäßig um den Strömungskanal (76) des Einstickansatzes verteilt sind und sich in Längsrichtung des Einsteckansatzes (24) erstrecken und wobei die Zwischenräume (112) zwischen den Randelementen (84) den Strömungskanal (70, 76) des Einsteckansatzes mit dem Strömungskanal (36, 40) des Hohlansatzes in Fluidverbindung bringen, so daß Fluid an der Strömungsregulierscheibe (50) verbeiströmen kann, wenn die Scheibe (50) auf den Randelementen (84) aufliegt,

dadurch gekennzeichnet, daß die Kupplungsteile (32, 62) zylindrisch geformt sind, daß an dem Einstickansatz (24) eine Querrippe mit einer Scheibenanlagefläche (130) angebracht ist, die verhindert, daß die Scheibe (50) sich in den Strömungskanal (70, 76) des Einsteckansatzes hineinbewegt und die sich über den Strömungskanal (70, 76) des Einsteckansatzes erstreckt und daß der Einsteckansatz (24) an der Grenze zwischen Kupplungsteil (62) und Schaftteil (60) von einer radialen abgeschrägten Dichtschulter (64) umgeben ist, die mit einer angepaßten Endfläche des Hohlansatzes (22) durch eine Ultraschallschweißung (26) verbunden ist.

2. Rückschlagventil nach Anspruch 1, bei dem die Bypass-Strömungswege (112) der Bypass- und Auflagermittel (120) im Querschnitt gewölbt sind.

3. Rückschlagventil nach Anspruch 1 oder 2, bei dem der Hohlansatz (22) einen Schaftteil (30) aufweist, in dem Schaftteil (30) und dem Kupplungsteil (32) jeweils eine durchgehende Bohrung (36, 40) ausgebildet sind und zwischen den Bohrungen (36, 40) eine Schulter (42) angeordnet ist, durch die sich eine Öffnung (46) erstreckt, welche die Bohrungen (36, 40) des Hohlansatzes miteinander in Fluidverbindung bringt.

4. Rückschlagventil nach den Ansprüchen 1 bis 3, bei dem in dem Schaftteil (60) und dem Kupplungsteil (62) des Einsteckansatzes (24) jeweils eine durchgehende Bohrung (70, 76) ausgebildet sind und zwischen den Bohrungen (70, 76) des Einsteckansatzes eine zweite Schulter (78) angeordnet ist, durch die sich eine Öffnung (80) erstreckt, welche die Bohrungen (70, 76) des Einsteckansatzes miteinander in Fluidverbindung bringt.

5. Rückschlagventil nach Anspruch 3 und 4, bei dem die Bohrungen (36, 70) der Ansatz-Schaftteile konisch verlaufen und die Bohrungen (40, 76) der Kupplungsteile im wesentlichen zylindrisch sind.

6. Rückschlagventil nach Anspruch 1, bei dem an dem Einsteckansatz eine abgeschrägte Partie (86) ausgebildet ist.

7. Rückschlagventil nach den Ansprüchen 1 bis 6, bei dem die Scheibe (50) ein spezifisches Gewicht von weniger als 1,0, vorzugsweise 0,98 aufweist.

8. Rückschlagventil nach den Ansprüchen 1 bis 7, bei dem die Scheibe (50) ausgestanzt ist.

9. Rückschlagventil nach den Ansprüchen 1 bis 8, dadurch gekennzeichnet, daß mit dem Hohlansatz (22) ein Strömungsschlauch (12) verbunden ist und daß an den Einsteckansatz (24) ein Y-Injektionseinrichtung (10) in Strömungsverbindung angeschlossen ist.

## Revendications

1. Clapet de non-retour (20) pour un dispositif de transfusion intraveineuse de liquides médicaux comprenant:

— un moyen femelle (22) en matériau polymère présentant un passage d'écoulement (36, 40) qui y est ménagé, un siège de soupape (42) qui y est ménagé et une partie d'accouplement (32) située sur lui;

— un moyen mâle (24) en matériau polymère présentant un passage d'écoulement (70, 76) qui y est ménagé, une partie tronconique (60) et une partie d'accouplement (62) portée par celle-ci, lesdits passages d'écoulement (36, 40, 70, 76) étant disposés de telle sorte qu'une communication de fluide puisse s'établir entre eux; les parties d'accouplement de moyen (32, 62) étant emboîtées l'une dans l'autre;

— un disque de commande d'écoulement circulaire (50) à surface supérieure et inférieure plates et bien dégagées monté mobile à l'intérieur dudit passage d'écoulement de moyeu femelle (40) et pouvant passer l'une première position d'appui sur ledit siège de soupape (42) obturant ledit passage d'écoulement de moyeu femelle (36) pour empêcher le retour du fluide dudit passage d'écoulement de moyeu mâle (70, 76) audit passage d'écoulement de moyeu femelle (36) à une seconde position dans laquelle le disque entier (50) est espacé du siège de soupape (42) permettant l'écoulement de fluide dudit moyeu femelle (22) audit moyeu mâle (24);

— des moyens de dérivation et de butée (120) prévus sur ledit moyeu mâle (24) coopérant avec le disque (50) étant espacés dudit siège de soupape (42) de sorte que ledit disque (50) fanchit un certain intervalle avant de buter contre lesdits moyens de dérivation et de butée (120), un passage d'écoulement (54) étant ménagé entre ledit disque (50) occupant sa seconde position et ledit passage d'écoulement de moyeu femelle (36, 40), lesdits moyens de dérivation et de butée (120) comportant une série d'éléments de jupe (84) angulairement espacées autour dudit passage d'écoulement de moyeu mâle (76) et s'étendant suivant la longueur dudit moyeu mâle (24), les espaces (112) séparant lesdits éléments de jupe (84) assurant une communication de fluide entre ledit passage d'écoulement de moyeu mâle (70, 76) et ledit passage d'écoulement de moyeu femelle (36, 40), de sorte que du fluide puisse franchir en dérivation ledit disque de commande d'écoulement (50) quand ce disque (50) est en appui sur lesdits éléments de jupe (84), caractérisé en ce que les parties d'accouplement de moyeu (32, 62) sont de forme cylindriques, en ce qu'une nervure transversale munie d'une surface de butée de disque (130) destinée à empêcher ledit disque (50) de pénétrer dans ledit passage d'écoulement de moyeu mâle (70, 76) est fixée audit moyeu mâle (24) et s'étend en travers dudit passage d'écoulement de moyeu mâle (70, 76) et en ce qu'un épaulement d'étanchéité conique radial (64) entoure le moyeu mâle (24) près de l'intersection de la partie d'accouplement (62) et de la partie tronconique (60), ledit épaulement d'étanchéité (64) étant relié à une surface d'extrémité conjuguée dudit moyeu femelle (22) par une soudure par ultrasons (26).

2. Clapet de non-retour selon la revendication 1, dans lequel les trajets d'écoulement dérivés (112) des moyens de dérivation et de butée (120) sont à section transversale incurvée.

3. Clapet de non-retour selon les revendications 1 ou 2, dans lequel ledit moyeu femelle (22) comporte encore une partie tronconique (30), la partie tronconique (30) et la partie d'accouplement (32) présentant chacune un trou (36, 40) qui y est ménagée, et un épaulement (42) situé entre lesdits trous (36, 40), ledit épaulement (42) présentant un orifice (46) qui le traverse pour faire communiquer ensemble lesdits trous de moyeu femelle (36, 40).

4. Clapet de non-retour selon l'une quelconque des revendications 1 à 3, dans lequel la partie tronconuque (60) et la partie d'accouplement (62) du moyeu mâle (24) présentant chacune un trou (70, 76) qui y est ménagé et un second épaulement (78) situé entre lesdits trous de moyeu mâle (70, 76), ledit second épaulement (78) présentant un orifice (80) qui le traverse pour faire communiquer ensemble les trous de moyeu mâle (70, 76).

5. Clapet de non-retour selon les revendications 3 et 4 dans lequel lesdits trous des parties tronconiques (36, 70 sont tronconiques et lesdits trous des parties d'accouplement (40, 76) sont sensiblement cylindriques.

6. Clapet de non-retour selon la revendication 1, dans lequel ledit moyeu mâle présente encore un tronçon tronconique (86).

7. Clapet de non-retour selon l'une quelconque des revendications 1 à 6, dans lequel ledit disque (50) a une densité inférieure à 1,0 et de préférence de 0,98.

8. Clapet de non-retour selon l'une quelconque des revendications 1 à 7, dans lequel ledit disque (50) est découpé à l'emporte-pièce.

9. Clapet de non-retour selon l'une quelconque des revendications 1 à 8, caractérisé en ce qu'un tube d'écoulement (12) est relié audit moyeu femelle (22) et à un raccord d'injection en Y (10)) assurant la communication en fluide avec ledit moyeu mâle (24).

0 075 039

FIG.1

FIG.2

FIG.3

FIG.4